Europäisches Patentamt

European Patent Office

Office européen des brevets

(11) Publication number: **0 201 348**
**A1**

(19)

(11) Publication number: **0 201 348 A1**

(12)

# EUROPEAN PATENT APPLICATION

(21) Application number: **86303551.5**

(22) Date of filing: **09.05.86**

(51) Int. Cl.⁴: **A 61 F 13/20, A 61 B 19/00**

(30) Priority: **10.05.85 JP 68179/85 U**

(43) Date of publication of application: **12.11.86**
**Bulletin 86/46**

(84) Designated Contracting States: **BE CH DE FR GB IT LI LU NL**

(71) Applicant: **TOKYO HAMAPUREN KABUSHIKI KAISHA, 121 Futatsuhashi-cho Seya-ku, Yokohama-shi Kanagawa-ken (JP)**

(72) Inventor: **Senya, Takeo, 2-16-21 Noshichiri Totsuka-ku, Yokohama-shi Kanagawa-ken (JP)**
Inventor: **Maeda, Kenji, 4150-92 Seya-cho Seya-ku, Yokohama-shi Kanagawa-ken (JP)**
Inventor: **Kawanishi, Yasuhiro, 265-105 Awaomadani, Minoo-shi Osaka-fu (JP)**

(74) Representative: **Senior, Alan Murray et al, J.A. KEMP & CO 14 South Square Gray's Inn, London WC1R 5EU (GB)**

(54) **Pads for surgical operations.**

(57) A pad for use in a surgical operation which has a body (1) of absorbent sponge-like material such as polyurethane foam and a ribbon (2) inserted through the body. The ribbon has sewn thereon a strand of fine wire (3) effective to shield X-rays, so that the pad can be detected by radiography, if it has inadvertently been left in a patient's abdomen.

EP 0 201 348 A1

- 1 -

## PADS FOR SURGICAL OPERATIONS

The present invention relates to pads for use in a surgical operation and more particularly, it relates to such pads for use in an abdominal cavity of a patient so as to retain the target and the surrounding organs in place and to arrest and absorb hemorrhage during the operation.

In general, a number of rolled gauzes are used in a surgical operation and introduced into an abdominal cavity of a patient to retain the internal organ to be operated and to arrest hemorrhage during the operation. While gauzes have generally been successful in retaining the internal organs, they have often created one or more problems negatively affecting the suitability of the gauzes for use in surgical operations. First, some one hundred gauzes are required to operate on a patient, for example, for gastric resection. Second, such gauzes lack firmness and are sometimes awkward to retain the internal organs. Third, they do not have a large capacity to absorb blood. Fourth, should one or more gauzes inadvertently be left in the patient's body, they can not be detected at all after an operation is finished.

Various attempts have been made heretofore to overcome the problems encountered with currently available gauzes. For example, Japanese Utility Model Publication No. 56-45605 discloses a pad for use in a surgical operation which comprises a body of a relatively high shape-retentive polyurethane foam and a plurality of thin strings attached thereto. Each of the strings has a weight attached to the tip thereof to facilitate removal of the pad from a patient's abdomen. While this pad generally successfully avoids the problems noted above, it has often created a problem that one or more strings tend to come off from the body, thereby leaving the pad in the patient's abdomen. Another disadvantage of this pad is that if it is inadvertently left in the patient's abdomen, it can not be detected after an operation has been finished.

Japanese Laid-Open Utility Model Publication No. 58-131809 discloses a modified pad in which a string is inserted through a body, one end of the string being knotted at one end of the body and the other end extending a substantial length from the other end of the body. However, this pad still entails the same disadvantage in that it may not be detected if inadvertently left in the patient's abdomen.

It is accordingly an object of the present invention to provide a novel pad for use in a surgical operation which reduces the above enumerated problems.

According to the present invention, there is provided a pad for use in a surgical operation comprising a body of absorbent sponge-like material, characterised by a ribbon inserted through and extending a substantial length from the opposite ends of said body, and in that said ribbon includes a strand of fine wire effective to shield X-rays.

With the invention, there is provided a pad which may positively retain an internal organ to be operated, thereby enhancing ease of operation, while eliminating or reducing the need for gauzes, and which may be reused, thereby minimising labour and costs in conjunction with surgical operations. Further, the pad may be detected by radiography if it has inadvertently been left in a patient, because the ribbon has sewn thereon a strand of fine wire effective to shield X-rays. Preferably, the absorbent sponge-like material forming the body is polyurethane foam, and the strand of fine wire sewn on the ribbon is a strand of stainless steel fibre.

In an alternative form of the invention, the body may be separated into two members.  One of the members is a shell member of a relatively soft polyurethane foam with a low specific gravity.  The other member is a core member of a relati.ely high shape-retentive polyurethane foam with a high specific gravity.  The pad thus constructed provides added softness to touch or better fitness to the internal organs.

The present invention will be more clearly understood from the following description given by way of example only with reference to the accompanying drawings, in which:

FIG. 1 is a perspective view of a pad according to the present invention;

FIG. 2 is an enlarged perspective view of a portion of the ribbon having a strand of fine wire sewn thereon; and

FIG. 3 is a perspective view, with portions broken away for clarity, of another embodiment of the invention.

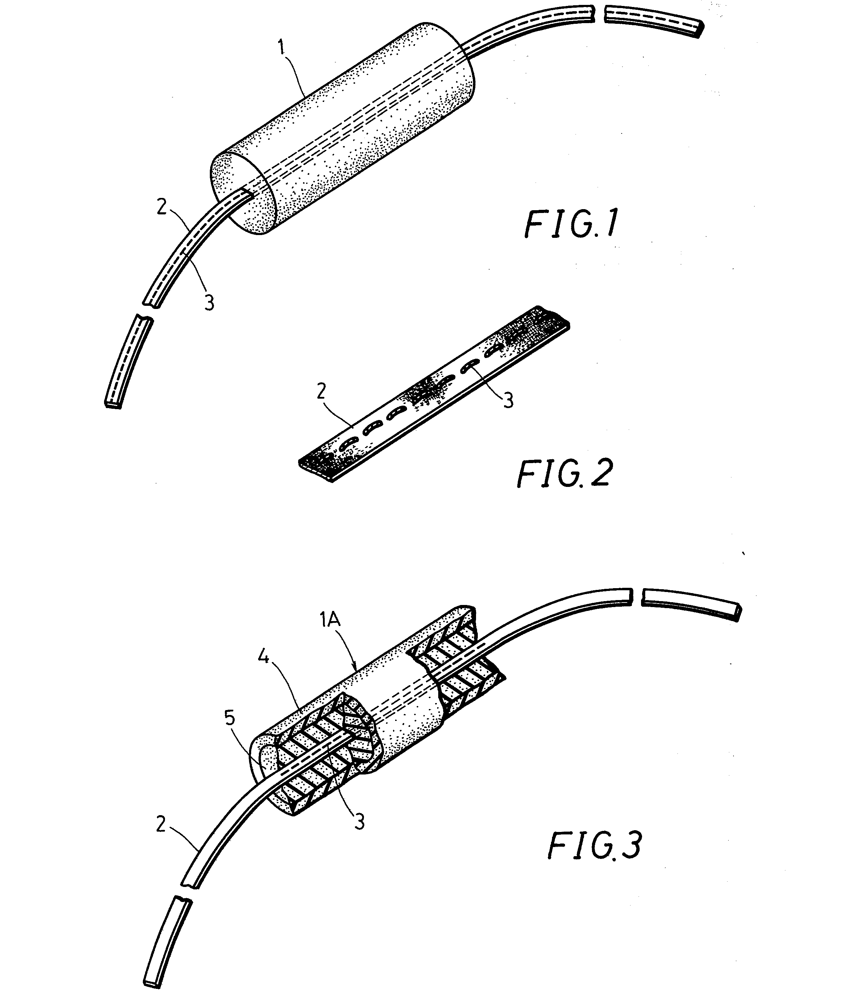

Referring to FIG. 1, shown therein is a pad constructed in accordance with the invention.  As shown therein, the pad includes a body 1 and a ribbon 2 inserted through the body 1. The body 1 may be of a suitable, absorbent sponge-like material such as polyurethane foam.  The ribbon 2 has sewn thereon a strand 3 of fine wire effective to shield X-rays.

While the elements of the invention are reduced in FIG. 1, it will be understood that the size of the body 1 is about 70 mm in diameter by about 180 mm in length.  The size of the body 1 may be larger or smaller than that described, depending on the purposes intended.  It will be also understood that the body 1 may be cylindrical, rectangular parallelepipedonal or any other shape suitable for the purposes intended.

The ribbon 2 is preferably of cotton cloth having a width of about 9 mm.  The length of the ribbon 2 is such that its opposite ends may be led out a substantial length from the abdomen of a patient (considered when in use).  This feature enables the pad to be readily taken out of the patient's body by pulling the opposite ends of the ribbon 2.

The strand 3 is made up of a plurality of fine, non-greasy stainless steel fiber having a diameter of 8 to 15 microns and is sewn on the ribbon 2 centrally in its longitudinal direction. Such stainless steel fibers are commercially available, and one type of such stainless steel fibers is produced by Tokyo Steel Co., Ltd. under the trade name "Susmic Fiber" (made of an austenitic stainless steel as described in JIS designation SUS 316L). While in FIG. 1 the strand 3 is shown as being sewn on the entire length of the ribbon 2, it will be understood that the strand 3 may be sewn only on a part of the ribbon 2 which is located within the body 1 (FIG. 3).

In operation, several pads are introduced into the abdominal cavity of a patient to retain the organ to be operated and/or the adjacent organs and to arrest and absorb hemorrhage. When a surgical operation is being completed, the pads are taken out slowly from the patient's abdomen by holding the opposite ends of the ribbon 2, so that blood and exudate caught in the pad should not be squeezed out.

As described above, the pad of the invention includes a ribbon on which a strand of fine wire is sewn having X-ray shielding effect. Should a pad inadvertently be left in a patient's body, it may be detected by projecting X-rays (produced by a high voltage of not greater than 70 kV) on the operated region. Thus, the detectable feature of the pad enhances the safety of operation at all times.

FIG. 3 shows an alternative body 1A which may be used in place of the body 1 shown in FIG. 1. The body 1A is separated into two members. One of the members is a shell member 4 of a relatively soft polyurethane foam with a specific gravity of 0.016. The other member is a core member 5 of a relatively high shape-retentive polyurethane foam with a specific gravity of 0.020. This feature of the outer shell 4 provides added softness to touch or better fitness to the internal organs.

-6-

C L A I M S

1. A pad for use in a surgical operation comprising a body (1) of absorbent sponge-like material, characterised by a ribbon (2) inserted through and extending a substantial length from the opposite ends of said body, and in that said ribbon includes a strand of fine wire (3) effective to shield X-rays.

2. A pad according to claim 1 characterised in that said absorbent sponge-like material of the body (1) comprises polyurethane foam.

3. A pad according to claim 1 or 2 characterised in that said body (1) of absorbent sponge-like material is of substantially cylindrical configuration.

4. A pad according to claim 1, 2 or 3 characterised in that said strand of fine wire (3) comprises a strand of stainless steel fibre.

5. A pad according to claim 1, 2, 3 or 4 characterised in that said strand of fine wire (3) is sewn only on a part of said ribbon (2) which is located within said body (1).

6. A pad according to any preceding claim characterised in that said body (1) includes one member being a shell member (4) of a relatively soft polyurethane foam with a low specific gravity and the other member being a core member (5) of a relatively high shape-retentive polyurethane foam with a high specific gravity, said ribbon (2) with said strand sewn therein being inserted through said core member (5).

7. A pad according to claim 6 characterised in that said core member (5) is of cylindrical configuration, and said shell member (4) is of annular configuration adapted to enclose said core member (5).

8. A pad according to claim 6 or 7 characterised in that said shell member (4) of a relatively soft polyurethane foam has a specific gravity of 0.016, and said core member (5) of a relatively high shape-retentive polyurethane foam has a specific gravity of 0.020.

9. A pad according to claim 6, 7 or 8 characterised in that said strand of fine wire (3) is sewn only in a part of said ribbon (2) which is located within said core member (5).

FIG.1

FIG.2

FIG.3

## DOCUMENTS CONSIDERED TO BE RELEVANT

EP 86303551.5

| Category | Citation of document with indication, where appropriate, of relevant passages | Relevant to claim | CLASSIFICATION OF THE APPLICATION (Int. Cl.4) |
|---|---|---|---|
| A | US - A - 3 961 629 (RICHTER et al.) <br> * Claim; column 4, lines 31-33; fig. 11; column 10, lines 42-44 * | 1-3 | A 61 F 13/20 <br> A 61 B 19/00 |
| A | DE - A1 - 2 801 682 (THE KENDALL) <br> * Claims 1-3,6 * | 1,4 | |
| A | US - A - 4 477 256 (HIRSCH) <br> * Claims 1,8; column 1, lines 27-29 * | 1,3 | |
| D,A | JP - U - 58-131 809 (TOKYO HAMA-PUREN KOGYO KABUSHIKI KAISHA) <br> * Fig. * | | |

|  | TECHNICAL FIELDS SEARCHED (Int. Cl.4) |
|---|---|
|  | A 61 F <br> A 61 B <br> A 61 L |

The present search report has been drawn up for all claims

| Place of search | Date of completion of the search | Examiner |
|---|---|---|
| VIENNA | 14-08-1986 | FARNIOK |